# EUROPEAN PATENT APPLICATION

(11) **EP 4 792 790 A1**
(43) Date of publication of application: **19.08.2026**
(21) Application number: 26158751.3
(22) Date of filing: 16.02.2026
(51) Int. Cl.: A61F 11/00, A61H 21/00, A61M 1/00

(54) **AID FOR RELEASING THE EAR MEMBRANE IN THE FORM OF A COMPRESSIBLE CONTAINER**

(30) Priority: 14.02.2025 CZ 20250046
(71) Applicant: Johann, Jaromir, 46015 Liberec 16 (CZ)
(72) Inventor: JANATA, Jiri, 46015 Liberec (CZ)
(74) Representative: Kratochvil, Vaclav

(57) **Abstract**

An aid for releasing the ear membrane in the form of a compressible container (1), which has an internal volume of 0.0018 to 0.0021 l, is provided with a neck (4) with a face for placement against the ear membrane (3) and with a check valve (2) for expelling air into the free space and creating negative pressure on the ear membrane. In an advantageous embodiment, the neck (4) is in the shape of a conical bellows with 2 to 3 corrugations with a diameter of 7 to 12 mm and the entire container is made of silicone.

## Description

### Technical Field

The technical solution relates to an aid for releasing the ear membrane in the form of a compressible container, intended for rehabilitation of the ear membrane.

### Background Art

Various solutions for ear treatment are known from technical practice, in particular using overpressure or various cleaning methods. For example, there are balloons of large volume intended for insertion into the nostrils and for creating overpressure in the ears. Their internal volume is usually from 0.025 to 0.52 l. The existing solutions, by their design, do not allow safe release of the ear membrane, wherein the existing balloons are used only for creating pressure in the nose, and only exceptionally, without the possibility of adjusting the magnitude of pressure or possibly negative pressure.

Existing balloons are disclosed, for example, in documents CN 116920188 and US 2018250192, where a balloon with a large internal volume is used to create overpressure in the ear.

Furthermore, there are various devices for cleaning ears, disclosed for example in documents CN 210170285U, US 2006253087, CN 217548369U, US 6059803, JPH 07108031, US 6406484 and US 2018250192, which are not intended for generating negative pressure on the ear membrane, because they could damage it during more frequent use. Moreover, these are relatively complex devices that are invasive and use electric cells and complicated adjustment elements.

During the search it was found that in most cases these aids are not used due to undesirable sound effects, sensitivity to adjustment, dependence on electricity, the need to replace batteries, and their short service life. The existing solutions are demanding both in operation and in maintenance and, given that they contain a relatively large number of components, their service life is limited.

### Summary of the Invention

The above-mentioned drawbacks are largely eliminated by an aid for releasing the ear membrane in the form of a compressible container according to the present invention. Its essence lies in that the container has an internal volume of 0.0018 to 0.0021 l, is provided with a neck with a face for placement on the ear membrane and with a check valve for expelling air into the free space and creating negative pressure on the ear membrane.

The neck is advantageously in the shape of a conical bellows with 2 to 3 corrugations with a diameter of 7 to 12 mm.

The container is, in an advantageous embodiment, made of silicone.

The proposed container is compact and is made of a harmless material, such as children's pacifiers. It is provided with a vent valve. The container is simple to manufacture and is suitable for use mainly by elderly persons.

The aid is very safe and has no electrical components. It is easily cleanable by washing in warm water. It operates reliably, has no adjustment elements and, due to low acquisition costs, is also occasionally replaceable.

The proposed attachment or adapter is, as a bellows, in the shape of a truncated cone and, thanks to its corrugations, is easily deformable and flexible, which, also due to its sensitivity, ensures gentle movements of the ear membrane, thereby ensuring the safety of the entire container. Together with the used material, silicone, like the entire container, it forms a compact unit.

The check valve is of the same design as in children's pacifiers, that is, it unidirectionally releases air from the ear space, whereby, after releasing the container, the creation of the required negative pressure of 20 to 40 millibars is enabled, so that the rehabilitation is sufficient and safe. With regard to the compactness of the container and the required volume, the volume of the container is considered including the adapter. However, according to the required material, its thickness and possibly the corrugations of the bellows, the volume may also change slightly.

An indispensable advantage, due to the simplicity of manufacture, is also the acquisition price, which allows occasional replacement of the aid. Another advantage is the possibility of using the container preventively in younger users, even before minimal symptoms of hearing impairment occur.

Thanks to its specific volume and the placement of the container in the ear, this specific design sufficiently ensures safe use for rehabilitation of the ear membrane, for which the container is primarily intended.

The proposed device is not dependent on an external source, does not produce sudden - undesirable sound effects and the like. Another advantage is simple operation and safe use thanks to the check valve, but above all the specific volume. The device may also be used only occasionally and thus preventively before hearing problems begin.

By inserting the container into the ear space, air is expelled through the valve into the free space. Subsequent release of the flexible container creates negative pressure and thereby releases the ear membrane and restores its function. The proposed shape of the adapter ensures tightness of the container for creating negative pressure.

The aid is safe even for lay users, its maintenance is easy and it has a relatively long service life. An advantage is the possibility of its environmentally friendly disposal.

### Brief Description of the Drawings

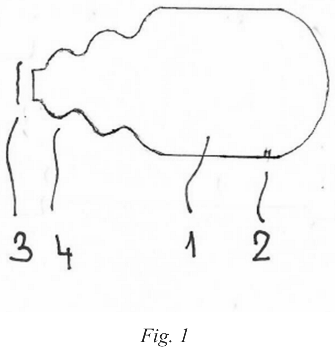

The aid for releasing the ear membrane according to this invention will be described in more detail using a specific example of an embodiment with the aid of the attached drawing, where in Fig. 1 an exemplary aid is schematically shown in a side view.

### Embodiments of the Invention

An exemplary aid for releasing the ear membrane is in the shape of a compressible container 1. The container 1 has an internal volume of 0.0019 l and is provided with a neck 4 with a face for placement on the ear membrane 3 and with a check valve 2 for expelling air into the free space and creating negative pressure on the ear membrane. The neck 4 is in the shape of a conical bellows with 2 corrugations with a diameter of 9 mm. The container 1 is made of silicone.

In a further embodiment, the container 1 has an internal volume of 0.002 l, the neck 4 is provided with three bellows and has a diameter of 12 mm.

By compressing the container 1, air is expelled through the valve 2 into the free space. After inserting the container 1 into the ear space near the ear membrane 3 and subsequently releasing the flexible container 1, negative pressure is created and thereby the ear membrane is released 3 and its function is restored. The proposed shape of the neck 4 in the form of an adapter ensures tightness of the container 1 in the ear for creating the required negative pressure.

The proposed aid, with its specific volume, is completely different from other devices for cleaning ears and aids for creating overpressure by inserting them into the nostril.

The ear membrane 3 is permanently exposed to atmospheric pressure between 950 and 1050 millibars. The proposed aid, by its design, makes it possible to achieve a value of 20 to 40 millibars, which is completely safe for the ear membrane 3 and ideal for its rehabilitation.

The exemplary aid is 25 mm long and its diameter is 12 mm, wherein the diameter of the neck is 7 mm.

### Industrial Applicability

The aid according to this invention will find application primarily in households, but also in hospitals, rehabilitation institutes, retirement homes and the like.

## Claims

1. An aid for releasing an ear membrane in the form of a compressible container (1), ***characterized in that*** the compressible container (1) has an internal volume of 0.0018 to 0.0021 l, the compressible container (1) is provided with a neck (4) with a face for placement against an ear membrane (3) and with a check valve (2) for expelling air into the free space and creating negative pressure on the ear membrane.

2. The aid according to claim *1, **characterized in that*** the neck (4) is in the shape of a conical bellows with 2 to 3 corrugations with a diameter of 7 to 12 mm.

3. The aid according to claim 1 or 2, ***characterized in that*** the compressible container (1) is made of silicone.
